(19) Europäisches Patentamt
European Patent Office
Office européen des brevets

(11) EP 0 983 768 A1

(12) # EUROPEAN PATENT APPLICATION
published in accordance with Art. 158(3) EPC

(43) Date of publication:
**08.03.2000 Bulletin 2000/10**

(21) Application number: **98921742.7**

(22) Date of filing: **20.05.1998**

(51) Int. Cl.[7]: **A61K 45/00**, A61K 31/70, A61K 31/44

(86) International application number:
**PCT/JP98/02223**

(87) International publication number:
**WO 98/52611 (26.11.1998 Gazette 1998/47)**

(84) Designated Contracting States:
**BE CH DE ES FR GB IT LI NL**

(30) Priority: **23.05.1997 JP 13348097**
**17.07.1997 JP 19255597**

(71) Applicant:
**Nippon Shinyaku Co., Ltd.**
**Kyoto-shi Kyoto 601-8550 (JP)**

(72) Inventors:
- **OZAKI, Takayuki**
  **Moriyama-shi, Shiga 524-0012 (JP)**
- **HIRATA, Yoshihisa**
  **Nagaokakyo-shi, Kyoto 617-0853 (JP)**
- **TADA, Shin-ichi**
  **Ohmihachiman-shi, Shiga 523-0022 (JP)**

(74) Representative:
**Strych, Werner Maximilian Josef, Dr. et al**
**Hansmann & Vogeser,**
**Patent- und Rechtsanwälte,**
**Albert-Rosshaupter-Strasse 65**
**81369 München (DE)**

(54) **MEDICINAL COMPOSITION FOR PREVENTION OR TREATMENT OF HEPATOPATHY**

(57) A medicinal composition containing an adenosine $A_2$ receptor agonist as the active ingredient. It is effective in the prevention or treatment of hepatopathy.

EP 0 983 768 A1

## Description

### TECHNICAL FIELD

The present invention relates to a pharmaceutical composition for prophylaxis or therapy of hepatic damage which comprises an adenosine $A_2$ receptor agonist as an active ingredient.

### BACKGROUND ART

The liver is injured by viruses, drugs, alcohol, and other factors to develop acute hepatitis, chronic hepatitis, fulminant hepatitis, and hepatic cirrhosis.

The current therapy of hepatic damage includes a general therapy aimed at increasing chances for healing, which is based on rest cure and dietary therapy supplemented by the administration of hepatoprotectants and, when viral infections are implicated, an antiviral therapy directed to inhibition of replication of the virus and an immunopotentiator therapy for potentiating the declined cellular immunity of the host. The hepatoprotectants which are traditionally used are liver hydrolysate, glycyrrhizin, reduced glutathione, tiopronin, polyene phosphatidylcholine, and so forth. The antiviral agent in use includes interferons, arabinosyladenine (Ara-A), arabinosyladenine monophosphate (Ara-AMP), acyclovir, and so forth. As the immunomodulator therapy, the use of glucocorticoids, interleukin 2, picibanil (OK-432), cianidanol, levamisole, etc. is being attempted. Interferons have both antiviral and immunomodulating activities. Prostaglandin E is known to have cytoprotective activity and, therefore, is expected to be of use for the protection of liver cells. Aside from them, human epidermal growth factor (hEGF) and human hepatocyte growth factor (hHGF) are reported to have cell growth-stimulating activity, thus being expected to find clinical application as a stimulator of liver regeneration, but are still in the stage of preclinical investigation. Recently, vaccine therapy has been recommended for the therapy and prophylaxis of hepatitis B virus.

However, none of those therapeutic drugs are sufficiently effective against hepatic damage and a strong demand exists for the creation of therapeutic drugs aimed at hepatic damage.

Meanwhile, it is known that adenosine $A_2$ receptor agonists have blood pressure-lowering, platelet aggregation-inhibitory, coronary blood flow-increasing, cerebral circulation-increasing, brain protective, antiarteriosclerotic, antiallergic, and antipsychotic actions. However, it is not known that those agonists ever show a hepatic damage inhibitory action.

### DISCLOSURE OF THE INVENTION

The object of the present invention is to provide an effective pharmaceutical composition for the prophylaxis or therapy of hepatic damage.

In view of the delayed development of drugs for the prophylaxis or therapy of hepatic damage, the inventors of the present invention scrutinized a variety of compounds and found unexpectedly that adenosine $A_2$ receptor agonists are useful for accomplishing the above object. The present invention has been completed on the basis of the above finding.

The present invention is characterized by the discovery that adenosine $A_2$ receptor agonists which are known to have blood pressure-lowering, platelet aggregation-inhibitory activities and so on have hepatic damage inhibitory activity which has not been heretofore recognized.

Referring to the active ingredient of the pharmaceutical composition of the invention, any adenosine $A_2$ receptor agonist can be used in the form of a pharmaceutical composition for the prophylaxis or therapy of hepatic damage according to the invention. Typical examples are adenosine $A_2$ receptor agonists having the following formula [1] with meeting the definition (a) or (b) below, their ester derivatives in which the free hydroxyl group or groups are esterified in a pharmaceutically acceptable form, and salts of said compounds or ester derivatives [hereinafter referred to collectively as adenosine $A_2$ receptor agonist of the invention].

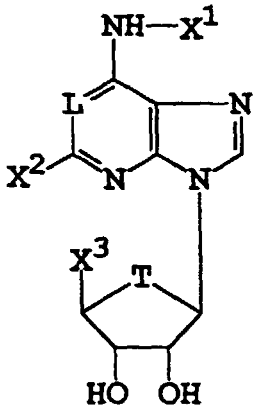

[1]

(a) Referring to formula [1], L represents N; T represents O; $X^1$ represents (1) hydrogen, (2) alkyl, (3) cycloalkyl, (4) aralkyl, (5) diarylalkyl (wherein one of the aryl moieties may be substituted by 1-3 same or different species of the substituent group $Y^8$ and the other aryl moiety being optionally substituted by 1-3 same or different species of the substituent group $Y^9$), or (6) arylheteroarylalkyl (wherein the aryl moiety may be substituted by 1-3 same or different species of the substituent group $Y^8$ and the heteroaryl moiety of which may be substituted by 1-3 same or different species of the substituent group $Y^9$); said substituent groups $Y^8$ and $Y^9$ are respectively selected from the group consisting of alkyl, halogen, alkoxy, alkylthio, trifluoromethyl, and tetramethylene;
$X^2$ represents hydrogen or any of the following substituent groups [i]-[iii]

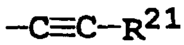

[i]

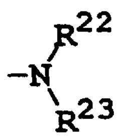

[ii]

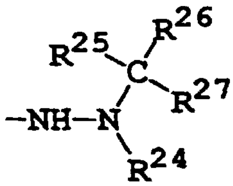

[iii]

wherein $R^{21}$ represents (1) cyano, (2) acyl, (3) carboxy, (4) alkoxycarbonyl, (5) alkenyl, (6) alkynyl, (7) aryl, (8) azido, (9) cycloalkyl which may be substituted by hydroxy, or (10) alkyl which may be substituted by the substituent $Y^1$ (the substituent $Y^1$ represents hydroxy, alkoxy, amino, carboxy, alkoxycarbonyl, alkenyl, alkynyl, aryl, azido, cyano or amino substituted by 1 or 2 same or different alkyl);
$R^{22}$ represents hydrogen or alkyl;
$R^{23}$ represents (1) cycloalkyl, (2) (cycloalky)alkyl wherein the cycloalkyl moiety may be substituted by the substituent $Y^2$ [the substituent $Y^2$ represents alkyl, hydroxy, alkylcarbonyloxy, alkoxy, or alkylenedioxy (wherein the two oxygen atoms are bound to either the same carbon atom or vicinal carbon atoms)], (3) aryl which may be substituted by 1-3 same or different species of the substituent group $Y^3$, (4) aralkyl (wherein the aryl moiety may be substituted by 1-3 species of the substituent group $Y^3$ and the alkyl moiety may be substituted by hydroxy), (5) arylcycloalkyl (wherein the aryl moiety may be substituted by 1-3 same or different species of the substituent group $Y^3$), (6) heteroaryl which may be substituted by 1-3 same or different species of the substituent group $Y^3$, (7) heteroarylalkyl (wherein the heteroaryl moiety may be substituted by 1-3 same or different species of the substituent group $Y^3$), (8) 9-fluorenyl, (9) 9-fluorenylalkyl, (10) 2-norbornyl, (11) bicycloheptylalkyl (wherein the bicycloheptyl moiety may be substituted by alkyl), (12) bicycloheptenylalkyl (wherein the bicycloheptenyl moiety may be substituted by alkyl), (13) adamantylalkyl, (14) cyclohexenylalkyl (wherein the cyclohexenyl moiety may be substituted by alkyl), (15) tetrahydropyranylalkyl, (16) tetrahydrothiopyranylalkyl, or
(17) the following substituent group,

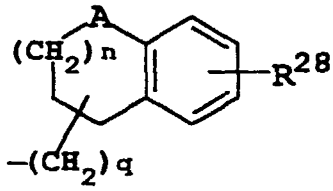

wherein A represents methylene, oxygen, or sulfur; n represents 0 or 1; q represents an integer of 0-2, $R^{28}$ represents hydrogen, alkyl, alkoxy, halogen or the substituent group -W-Z defined hereinafter.

The substituent group $Y^3$ is selected from the group consisting of (1) alkyl, (2) alkoxy, (3) alkylthio, (4) alkylcarbonyloxy, (5) halogen, (6) hydroxy, (7) benzyloxy (wherein the benzene moiety may be substituted by halogen), (8) tri-fluoromethyl, and (9) the substituent group -W-Z.

W represents (1) a single bond, (2) alkylene, (3) alkenylene, (4) thioalkylene, or (5) oxyalkylene. Z represents (1) cyano, (2) carboxy, (3) alkoxycarbonyl, (4) carbamoyl, (5) carbamoyl substituted by 1-2 same or different species of alkyl, or (6) carbamoyl substituted by alkyl which is substituted by $NHR^{29}$ ($R^{29}$ represents hydrogen, alkyl, alkenyl, acyl, or isothiocyano).

$R^{25}$, $R^{26}$, and $R^{27}$ may be the same or different and each represents hydrogen, alkyl, cycloalkyl, or aryl which may be substituted by the substituent group $Y^5$. The substituent group $Y^5$ represents alkyl, alkoxy, nitro, or amino which may be substituted by alkyl or phenyl. $R^{24}$ represents hydrogen or alkyl; or $R^{24}$ and $R^{27}$ taken together represent a bond.

$X^3$ represents hydroxymethyl, alkoxymethyl, aminomethyl, azidomethyl, cyanomethyl, carboxy, alkoxycarbonyl, cycloalkyloxycarbonyl, $-CON(R^{32})(R^{33})$, $-CH_2NHSO_2R^{34}$, $-CH_2NHC(=O)R^{35}$, or $-CH_2NHC(=S)R^{36}$.

$R^{32}$ and $R^{33}$ may be the same or different and each represents hydrogen, aralkyl, cycloalkyl, or alkyl which may be substituted by hydroxy. $R^{34}$ represents alkyl or aryl which may be substituted by alkyl. $R^{35}$ and $R^{36}$ each represents hydrogen, alkyl, amino, or alkylamino.

Provided, however, that at least one of $X^1$ and $X^2$ represents hydrogen.

(b) Referring to formula [1], L represents N, N→O, or CH; T represents $CH_2$ or O; $X^1$ represents $-E-(G)_m-R^{50}$.

E represents (1) alkylene which may be substituted by 1-3 same or different species of $Y^6$, (2) cycloalkylene which may be substituted by 1-3 same or different species of $Y^6$, or (3) cycloalkenylene which may be substituted by 1-3 same or different species of $Y^6$. The substituent $Y^6$ is selected from the group consisting of alkyl, halogen, and alkoxy.

G represents $NR^{54}$, oxygen, or sulfur; m represents 0 or 1.

$R^{54}$ represents hydrogen, alkyl, aryl, or heteroaryl.

$R^{50}$ represents heteroaryl which may be substituted by 1-3 same or different species of $Y^7$. $Y^7$ represents (1) hydrogen, (2) hydroxy, (3) alkyl which may be substituted by hydroxy, (4) alkylthio, (5) mercaptoalkyl, (6) alkoxy, (7) alkoxyalkyl, (8) amino which may be substituted by alkyl, (9) carboxy, (10) acyl, (11) halogen, (12) carbamoyl which may be substituted by alkyl, (13) aryl, or (14) heteroaryl.

$X^2$ represents hydrogen.

$X^3$ represents $-CON(R^{51})(R^{52})$ or $-CH_2OR^{53}$.

$R^{51}$, $R^{52}$, and $R^{53}$ may be the same or different and each represents hydrogen, alkyl, aryl, or heteroaryl.

The present invention is now described in detail.

With reference to the adenosine $A_2$ receptor agonist of the present invention, the "alkyl" includes straight-chain or branched alkyl groups of 1-16 carbon atoms such as methyl, ethyl, n-propyl, isopropyl, n-butyl, isobutyl, sec-butyl, tert-butyl, n-pentyl, isopentyl, n-hexyl, isohexyl, n-heptyl, isoheptyl, n-octyl, isooctyl, n-nonyl, isononyl, n-decyl, isodecyl, n-undecyl, isoundecyl, n-dodecyl, isododecyl, n-tridecyl, isotridecyl, n-tetradecyl, isotetradecyl, n-pentadecyl, isopentadecyl, n-hexadecyl, and isohexadecyl.

The alkyl moiety of said "alkylcarbonyloxy", "alkoxyalkyl", "alkylthio", "mercaptoalkyl", "(cycloalkyl)alkyl", "aralkyl", "diarylalkyl", "heteroarylalkyl", "arylheteroarylalkyl", "9-fluorenylalkyl", "bicycloheptylalkyl", "bicycloheptenylalkyl", "adamantylalkyl", "cyclohexenylalkyl", "tetrahydropyranylalkyl", "tetrahydrothiopyranylalkyl", "alkoxyalkyl", "cycloalkyloxycarbonyl" or "alkylamino" includes the alkyl groups mentioned hereinbefore.

The "alkenyl" includes straight-chain or branched alkenyl groups of 2-16 carbon atoms such as vinyl, 1-propenyl, allyl, 1-butenyl, 2-butenyl, 3-butenyl, 1-pentenyl, 4-pentenyl, 1-hexenyl, 5-hexenyl, 1-heptenyl, 6-heptenyl, 1-octenyl, 7-octenyl, 1-nonenyl, 8-nonenyl, 1-decenyl, 9-decenyl, 1-undecenyl, 10-undecenyl, 1-dodecenyl, 11-dodecenyl, 1-tridecenyl, 12-tridecenyl, 1-tetradecenyl, 13-tetradecenyl, 1-pentadecenyl, 14-pentadecenyl, 1-hexadecenyl, and 15-hexadecenyl.

The "alkynyl" includes straight-chain or branched alkynyl groups of 2-16 carbon atoms such as ethynyl, 1-propynyl, 2-propynyl, 1-butynyl, 2-butynyl, 3-butynyl, 1-pentynyl, 4-pentynyl, 1-hexynyl, 5-hexynyl, 1-heptynyl, 6-heptynyl, 1-octynyl, 7-octynyl, 1-nonynyl, 8-nonynyl, 1-decynyl, 9-decynyl, 1-undecynyl, 10-undecynyl, 1-dodecynyl, 11-dodecynyl, 1-tridecynyl, 12-tridecynyl, 1-tetradecynyl, 13-tetradecynyl, 1-pentadecynyl, 14-pentadecynyl, 1-hexadecynyl, and 15-hexadecynyl.

The "alkylene" includes straight-chain or branched alkylene groups of 1-16 carbon atoms such as methylene, ethylene, trimethylene, tetramethylene, pentamethylene, hexamethylene, heptamethylene, octamethylene, nonamethylene, decamethylene, undecamethylene, dodecamethylene, tridecamethylene, tetradecamethylene, pentadecamethylene, hexadecamethylene, propylene, ethylethylene, methyltrimethylene, methyltetramethylene, methylpentamethylene, methylhexamethylene, methylheptamethylene, methyloctamethylene, methylnonamethylene, methyldecamethylene, methylundecamethylene, methyldodecamethylene, methyltridecamethylene, methyltetradecamethylene, and methylpentadecamethylene, among others.

The alkylene moiety of said "thioalkylene", "oxyalkylene", or "alkylenedioxy" includes the alkylene groups mentioned above.

The "alkenylene" includes straight-chain or branched alkenylene groups of 2-16 carbon atoms such as vinylene, propenylene, butenylene, pentenylene, hexenylene, heptenylene, octenylene, nonenylene, decenylene, undecenylene, dodecenylene, tridecenylene, tetradecenylene, pentadecenylene, hexadecenylene, methylbutenylene, ethylbutenylene, methylpentenylene, methylhexenylene, methylheptenylene, methyloctenylene, methylnonenylene, methyldecenylene, methylundecenylene, methyldodecenylene, methyltridecenylene, methyltetradecenylene, and methylpentadecenylene, among others.

The "cycloalkyl" includes cycloalkyl groups of 3-8 carbon atoms such as cyclopropyl, cyclobutyl, cyclopentyl, cyclohexyl, cycloheptyl, and cyclooctyl.

The cycloalkyl moiety of said "(cycloalkyl)alkyl", "arylcycloalkyl", or "cycloalkyloxycarbonyl" includes the cycloalkyl groups mentioned hereinbefore.

The "cycloalkylene" means a bivalent group available upon elimination of two hydrogen atoms from different carbon atoms of a cycloalkane of 3-8 carbon atoms, thus including cyclopropylene, cyclobutylene, cyclopentylene, cyclohexylene, cycloheptylene, and cyclooctylene.

The "cycloalkenylene" means a bivalent group available upon elimination of two hydrogen atoms from different carbon atoms of a cycloalkene of 4-8 carbon atoms, thus including cyclobutenylene, cyclopentenylene, cyclohexenylene, cycloheptenylene, and cyclooctenylene.

The "alkoxy" includes straight-chain or branched alkoxy groups of 1-16 carbon atoms such as methoxy, ethoxy, n-propoxy, isopropoxy, n-butoxy, isobutoxy, sec-butoxy, tert-butoxy, n-pentyloxy, isopentyloxy, n-hexyloxy, isohexyloxy, n-heptyloxy, isoheptyloxy, n-octyloxy, isooctyloxy, n-nonyloxy, isononyloxy, n-decyloxy, isodecyloxy, n-undecyloxy, isoundecyloxy, n-dodecyloxy, isododecyloxy, n-tridecyloxy, isotridecyloxy, n-tetradecyloxy, isotetradecyloxy, n-pentadecyloxy, isopentadecyloxy, n-hexadecyloxy, and isohexadecyloxy.

The alkoxy moiety of said "alkoxycarbonyl", "alkoxyalkyl", or "alkoxymethyl" includes the alkoxy groups mentioned hereinbefore.

The "bicycloheptyl" includes bicyclo[2.2.1]heptyl and bicyclo[3.1.1]heptyl such as 2-bornyl, 2-(2-neobornyl), 3-(2-isobornyl), 4-(2-norbornyl), and 5-(6,6-dimethylbicyclo-[3.1.1]heptan-2-yl), among others.

The bicycloheptyl moiety of said "bicycloheptylalkyl" includes the bicycloheptyl groups mentioned hereinbefore.

The "bicycloheptenyl" includes bicyclo[2.2.1]heptenyl and bicyclo[3.1.1]heptenyl such as norborn-5-en-2-yl and 2-(6,6-dimethylbicyclo[3.1.1]hept-2-en-2-yl) among others.

The bicycloheptenyl moiety of said "bicycloheptenylalkyl" includes the bicycloheptenyl groups mentioned hereinbefore.

The "aryl" includes aryl groups of 6-10 carbon atoms such as phenyl, 1-naphthyl and 2-naphthyl.

The aryl moiety of said "aralkyl" or "arylcycloalkyl" includes the above-mentioned aryl groups.

The "diarylalkyl" means alkyl substituted by two same or different aryl groups. The aryl moieties of "diarylalkyl" include the above-mentioned aryl groups. The alkyl moiety of said "diarylalkyl" includes the alkyl groups mentioned hereinbefore.

The "heteroaryl" includes aromatic 5- or 6-membered heterocyclic groups each containing 1-4 hetero-atoms which may be same or different members selected from among nitrogen, oxygen, and sulfur as ring atoms and the corresponding benzenoid (beuzene-fused) heterocyclic systems. As such, the heteroaryl includes but is not limited to pyridin-2-yl, pyridin-3-yl, pyridin-4-yl, pyrrol-2-yl, pyrrol-3-yl, thio-phen-2-yl, thiophen-3-yl, imidazol- 1-yl, pyrimidin-2-yl, pyrimidin-4-yl, pyrimidin-5-yl, 1,3-thiazol-2-yl, 1, 3-oxazol-2-yl, indol-2-yl, indol-3-yl, indol-4-yl, indol-5-yl, indol-6-yl, indol-7-yl, benzofuran-2-yl, benzofuran-3-yl, benzofuran-4-yl, benzofuran-5-yl, benzofuran-6-yl, benzofuran-7-yl, benzothiophen-2-yl, benzothiophen-3-yl, benzothiophen-4-yl, benzothiophen-5-yl, benzothiophen-6-yl, benzothiophen-7-yl, benzothiazol-2-yl, benzoisothioazol-3-yl, benzothiazol-4-yl, benzothiazol-5-yl, benzothiazol-6-yl, and benzothiazol-7-yl.

The heteroaryl moiety of said "heteroarylalkyl" includes the above-mentioned heteroaryl groups.

The "arylheteroarylalkyl" means alkyl substituted by aryl and heteroaryl. The aryl moiety of said "arylheteroarylalkyl" includes the above-mentioned aryl groups. The heteroaryl moiety of said "arylheteroarylalkyl" includes the above-mentioned heteroaryl groups. The alkyl moiety of said "arylheteroarylalkyl" includes the above-mentioned alkyl groups.

The "acyl" means "alkylcarbonyl" and "arylcarbonyl".

The alkyl moiety of "alkylcarbonyl" includes the above-mentioned alkyl groups. The aryl moiety of said "arylcarbonyl" includes the above-mentioned aryl groups. The "acyl" includes but is not limited to acetyl, propionyl, butyryl, valeryl, hexanoyl, heptanoyl, octanoyl, nonanoyl, decanoyl, lauroyl, myristoyl, palmitoyl, benzoyl, 1-naphthoyl, and 2-naphthoyl.

The "halogen" includes fluorine, chlorine, bromine, and iodine.

The "an ester derivative in which the free hydroxyl group or groups are esterified in a pharmaceutically acceptable form" means a compound which is available upon esterification of all or not less than one hydroxyl groups selected from among the two hydroxyl groups shown in formula [1] and the hydroxyl group or groups which may be possessed by the substituent groups $X^1$, $X^2$, and $X^3$ defined with regard to formula [1] and which is convertible to a free hydroxyl-containing compound of formula [1] under physiological conditions.

The ester derivative thus includes but is not limited to esters with straight-chain or branched alkanoic acids of 1-30 carbon atoms (esters with acetic acid, isobutyric acid, pivalic acid, octanoic acid, lauric acid, myristic acid, palmitic acid, stearic acid, etc.), esters with alkoxyalkanoic acids (esters with methoxyacetic acid, 3-ethoxypropionic acid, etc.), esters with arylcarboxylic acids (esters with benzoic acid, nicotinic acid, etc.), carbamic acid ester, and esters with mono- or dialkylcarbamic acids (e.g. esters with mono- or dimethylcarbamic acid).

As the adenosine $A_2$ receptor agonist which can be used as the active ingredient of the pharmaceutical composition of the invention, the following specific compounds can be mentioned.

(1) 1-[6-Amino-2-[(2-(4-(3-((2-aminoethyl)amino)-3-oxopropyl)phenyl)ethy])amino]-9H-purin-9-yl]-1-deoxy-N-ethyl-β-D-ribofuranuronamide (US 5,280,015)
(2) 2-[(2-Cyclohexylethyl)amino]adenosine (Japanese Laid-Open H1-265100)
(3) 2-[(2-(1-Cyclohexen-1-yl)ethyl)amino]adenosine (Japanese Laid-Open H1-265100)
(4) 2-(1-Octyn-1-yl)adenosine (Japanese Laid-Open S62-99395)
(5) 1-[6-Amino-2-[(2-(4-(2-carboxyethyl)phenyl)ethyl)amino]-9H-purin-9-yl]-1-deoxy-N-ethyl-β-D-ribofuranuronamide (Japanese Laid-Open S63-201196)
(6) 1-[6-Amino-2-[(2-(4-(2-carboxyethyl)phenyl)ethyl)amino]-9H-purin-9-yl]-1-deoxy-N-ethyl-β-D-ribofuranuronamide sodium salt (Japanese Laid-Open S63-201196)
(7) 1-[6-Amino-2-(1-hexyn-1-yl)-9H-purin-9-yl]-1-deoxy-β-D-ribofuranuronamide (Japanese Laid-Open H5-25195)
(8) 1-[6-Amino-2-(1-hexyn-1-yl)-9H-purin-9-yl]-1-deoxy-N-ethyl-β-D-ribofuranuronamide (Japanese Laid-Open H5-25195)
(9) 4-[7-((1-((3-Chloro-2-thienyl)methyl)propyl)amino)-3H-imidazo [4,5-b]pyridin-3-yl]-N-ethyl-2,3-dihydroxycyclopentanecarboxamide (WO 95/28160)
(10) 2-[((R)-1-Methyl-2-phenylethyl)amino]adenosine (WO 94/07905)
(11) 2-[(Cyclohexylmethylene)hydrazino]adenosine (Japanese Laid-Open H6-128281)
(12) 2-(Phenylamino)adenosine (Japanese Laid-Open S49-80096)
(13) $N^6$-[2-(3,5-Dimethoxyphenyl)-2-(2-methylphenyl)-ethyl]adenosine (WO 88/03147)
(14) 1-(6-Amino-9H-purin-9-yl)-1-deoxy-N-cyclopropyl-β-D-ribofuranuronamide (Japanese Laid-Open S49-13200)
(15) Ethyl 1-(6-amino-9H-purin-9-yl)-1-deoxy-β-D-ribofuranuronate (Japanese Laid-Open S47-3178)
(16) Adenosine (The Merck Index 11th edition, 143)

Among species of the adenosine $A_2$ receptor agonist according to the invention, compounds containing acidic groups can be used as they are in the free acid form or optionally in the form of pharmaceutically acceptable salts prepared by the per se known method, as drugs. As examples of the salt, there can be mentioned alkali metal salts, such as salts with sodium and potassium, and alkaline earth metal salts, such as salts with calcium.

Among species of the adenosine $A_2$ receptor agonist according to the present invention, compounds containing basic groups can be used as they are in the free base form or optionally in the form of pharmaceutically acceptable salts prepared by the per se known method, as drugs. The salt mentioned just above includes salts with mineral acids such as hydrochloric acid, hydrobromic acid, sulfuric acid and phosphoric acid, and salts with organic acids such as acetic acid, citric acid, tartaric acid, maleic acid, succinic acid, fumaric acid, p-toluenesulfonic acid, benzenesulfonic acid, and methanesulfonic acid.

The above compound of formula [1] and the ester derivative of compound [1] in which the free hydroxyl group or groups are esterified in a pharmaceutically acceptable form can be produced by any of the processes described in

Japanese Laid-Open H5-25195, Japanese Laid-Open H1-265100, Japanese Laid-Open S62-99395, and Japanese Laid-Open S63-201196, WO 94/07905, Japanese Laid-Open H6-128281, Japanese Laid-Open H5-9197, and Japanese Laid-Open H5-163294, US 5,280,015, WO 95/28160, WO 88/03147, Japanese Laid-Open S47-3178, Japanese Laid-Open S49-80096, or Japanese Laid-Open S49-13200 or a process analogous to any of the above-mentioned processes.

The adenosine $A_2$ receptor agonist according to the present invention has very excellent inhibitory activity against elevation of serum transaminase activities as can be seen from Test Examples which appear hereinafter and, therefore, can be used in the prophylaxis or therapy of hepatic damage. Since adenosine $A_2$ receptor agonists are not known to have antiviral activity, the prophylactic or therapeutic efficacy mentioned above may not be attributed to their antiviral action.

As the adenosine $A_2$ receptor agonist for use in the present invention, compounds with comparatively low toxicity at their prophylactically or therapeutically effective doses for hepatic damage should be chosen.

For administration of the adenosine $A_2$ receptor agonist as a prophylactic or therapeutic drug in accordance with the invention, the compound of the invention can be administered to animals inclusive of man, either as it is or in the form of a pharmaceutical composition containing 0.01%-99.5%, preferably 0.5%-90%, of the compound of the invention in a medicinally acceptable, nontoxic and inert carrier.

The carrier that can be used is one or more members selected from the group consisting of solid, semi-solid and liquid diluents, fillers, and other auxiliary agents. The pharmaceutical composition is preferably administered in unit dosage forms. The pharmaceutical composition of the invention can be administered by the orally, into the tissue, intravenously, topically (transdermally etc.), or rectally. Of course, dosage forms suited to the respective routes of administration are employed. Oral or intravenous administration, in particular, is preferred.

The dosage for the prophylaxis or therapy of hepatic damage is preferably selected according to patient factors such as age and body weight, route of administration, nature and severity of illness, and other factors. Usually, however, the effective dose in an adult human is generally 0.01-1000 mg/day and preferably 0.1-100 mg/day.

There may be cases in which lower doses are sufficient, while larger doses may be recommendable in other cases. The above daily dosage can be administered in 2-3 divided doses.

Oral administration can be carried out using solid or liquid unit dosage forms such as bulk powders, powders, tablets, dragees, capsules, granules, suspensions, solutions, syrups, drops and sublingual tablets.

Bulk powders can be manufactured by pulverizing the active compound to a suitable particle size. Powders can be manufactured by pulverizing the active compound to a suitable particle size and mixing it with a similarly pulverized pharmaceutical carrier such as edible carbohydrates, e.g. starch, mannitol, etc., and/or other suitable materials. Where necessary, a flavorant, preservative, dispersant, coloring agent, perfume, etc. can also be added.

Capsules can be manufactured by filling gelatin or other capsule shells with said bulk powders, the powders prepared as above, or the granules prepared by the procedure described below for tablets. Lubricants and/or fluidizing agents, such as colloidal silica, talc, magnesium stearate, calcium stearate, and solid polyethylene glycol, may be added in finely divided form prior to the filing operation described above. Disintegrators and solubilizers, such as carboxymethyl-cellulose, carboxymethylcellulose calcium, low-substitution hydroxypropylcellulose, croscarmellose sodium, carboxymethylstarch sodium, calcium carbonate, and sodium carbonate can also be added, in which case the efficacy of the drug after ingestion of the capsules may be enhanced.

The finely divided compound of the invention can be suspended and dispersed in vegetable oil, polyethylene glycol, glycerin, or a surfactant and packaged in a gelatin sheet to provide soft capsules. Tablets can be manufactured by preparing a powdery mixture of the compound with an excipient, processing it into granules or slags, adding a disintegrator and/or a lubricant, and compressing the mixture. The powdery mixture can be prepared by mixing adequately pulverized powders of the active compound with any of said diluents or bases. Where necessary, binders (e.g. carboxymethylcellulose sodium, methylcellulose, hydroxy-propylmethylcellulose, gelatin, polyvinylpyrrolidone, polyvinyl alcohol), dissolution retardants (e.g. paraffin), reabsorption agents (e.g. quaternary salts), and/or adsorbents (e.g. bentonite, kaolin, dicalcium phosphate) can also be added. The powdery mixture can be made into granules by wetting it with a binder, such as a syrup, a starch paste, gum arabic, a cellulose solution, or a polymer solution, stirring the wet powder well, drying it, and pulverizing the same. Instead of converting the powders to granules in the above manner, the powders may be compressed with a tablet machine and the resulting crude slags be comminuted into granules.

The granules thus prepared can be protected against conglomeration by adding a lubricant such as stearic acid, a salt of stearic acid, talc or mineral oil. The thus-lubricated composition is then compressed. The resulting core tablets can be coated with a film coating agent or a sugar coating agent.

As an alternative, the active compound can be directly mixed with a free-flowing inert carrier without being subjected to the above-mentioned granulation or slagging procedure and the mixture be directly compressed. A transparent or translucent protective coating capable of yielding a hermetic shellac or other film, a sugar coating, a polymer coating, or a glaze wax coating, for instance, can also be applied.

Other dosage forms for oral administration, such as solutions, syrups, or elixirs, can also be provided in unit

dosage forms each containing a predetermined amount of the drug. Syrups are manufactured by dissolving the active compound in a suitable flavored aqueous medium, while elixirs are manufactured using a nontoxic alcoholic vehicle. Suspension are prepared by dispersing the active compound in nontoxic vehicles. Where necessary, solubilizers and emulsifiers (e.g. ethoxylated isostearyl alcohol, polyoxyethylene sorbitol ester, etc.) as well as preservatives and flavorants (e.g. peppermint oil, saccharin, etc.) can also be added.

If necessary, unit dosage formulations for oral administration can be microencapsulated. Such formulations can also be coated with, or embedded in, a polymer or wax matrix for prolonged action or sustained release.

Parenteral administration can be carried out using liquid unit dosage forms, e.g. solutions or suspensions, for subcutaneous, intramuscular or intravenous injection. Such dosage forms can be manufactured by suspending or dissolving a predetermined amount of the active compound in an injectable nontoxic liquid vehicle, e.g. an aqueous medium or an oily medium, and sterilizing the resulting suspension or solution. To make an injection isotonic, a nontoxic salt or a solution thereof can be added. Moreover, stabilizers, preservatives, emulsifiers, and other additives can also be employed.

Rectal administration can be made using suppositories manufactured by dissolving or suspending the active compound in a low-melting water-soluble or water-insoluble solid medium, e.g. polyethylene glycol, cacao butter, a semi-synthetic oleaginous base (e.g. Witepsol™), a higher fatty acid ester (e.g. myristyl palmitate) or a mixture thereof.

BEST MODE FOR CARRYING OUT THE INVENTION

The following test examples and formulation examples are intended to illustrate the present invention in further detail and should by no means be construed as defining the scope of the invention.

In the test and formulation examples, compound 1 represents 1-[6-amino-2-[(2-(4-(2-carboxyethyl)phenyl)-ethyl)amino]-9H-purin-9-yl]-1-deoxy-N-ethyl-β-D-ribofuranuronamide; compound 2 represents 2-(phenylamino)adenosine; compound 3 represents $N^6$-[2-(3,5-dimethoxyphenyl)-2-(2-methylphenyl)ethyl]adenosine; compound 4 represents 1-(6-amino-9H-purin-9-yl)-1-deoxy-N-cyclopropyl-β-D-ribofuranuronamide; and compound 5 represents 2-(1-octyn-1-yl)adenosine.

Test Example 1

Inhibitory effect on concanavalin A-induced hepatic damage

The hepatic damage inhibitory effect of the adenosine $A_2$ receptor agonist of the invention can be verified by a test using mice as described below. Among the various animal models proposed for the evaluation of hepatic damage inhibitory activity, the disease model used in this test resembles human hepatitis in the mechanism of onset.

Using 6-weeks old male BALB/c mice in groups of 17-20, the test drug was administered intraperitoneally or orally. At 30 minutes after intraperitoneal administration of the test drug or 1 hour after oral administration, 0.6 mg of concanavalin A (con A) was administered intravenously to each animal to induce hepatic damage. At 24 hours after administration of con A, plasma transaminase activities [GOT (glutamic-oxaloacetic transaminase), GPT (glutamic-pyruvic transaminase)] were determined. The plasma transaminase activity inhibition rate in each group given a test drug relative to the control group was calculated by means of the following equation.

$$\text{Inhibition rate (\%)} = [(C-D)/C] \times 100$$

where C represents the mean value of plasma transaminase activity in the control group and D represents the mean value of plasma transaminase activity in the group given a test drug.

The results are presented in Table 1.

Table 1

| Inhibitory effects on concanavalin A-induced hepatic damage in mice | | | | | | |
|---|---|---|---|---|---|---|
| Test drug | n | Dosage (mg/kg) | Inhibition rate (%) | | | |
| | | | i.p. | | p.o. | |
| | | | GOT | GPT | GOT | GPT |
| Compound 1 | 20 | 0.3 | 85** | 88** | | |
| | 20 | 3 | 93** | 98** | 64* | 58* |
| Compound 2 | 19 | 0.3 | 61** | 65** | | |
| | 19 | 1 | 81** | 91** | | |
| Compound 3 | 17 | 0.3 | 61** | 60** | | |
| | 17 | 3 | 83** | 87** | 86** | 88** |
| Compound 4 | 18 | 1 | | | 49** | 56** |
| Compound 5 | 20 | 0.3 | 90** | 96** | | |
| | 20 | 3 | 91** | 96** | 93** | 97** |

*: $P<0.05$,
**: $P<0.01$

Whether administered intraperitoneally or administered orally, the adenosine $A_2$ receptor agonist of the invention inhibited elevation of plasma transaminase activities, which are markers of liver impairment, in significant measures. It is, therefore, clear that the adenosine $A_2$ receptor agonist of the invention has high hepatic damage inhibitory activity.

Test Example 2

Acute toxicity study

Using ICR mice, 7-8 weeks old, Compound 5 was administered orally in a dose of 1000 mg/kg. As a result, no death was encountered (Japanese Laid-Open H3-287537).

Formulation Example 1

Tablets (oral)

In each 120 mg per tablet

| | |
|---|---|
| Compound 1 | 1 mg |
| Lactose | 60 mg |
| Corn starch | 30 mg |
| Crystalline cellulose | 20 mg |
| Hydroxypropylcellulose | 7 mg |
| Magnesium stearate | 2 mg |

A powdery mixture of the above formulation is compressed to provide tablets.

Formulation Example 2

Tablets (oral)

In each 120 mg per tablet

| | |
|---|---|
| Compound 3 | 1 mg |
| Lactose | 60 mg |
| Corn starch | 30 mg |
| Crystalline cellulose | 20 mg |
| Hydroxypropylcellulose | 7 mg |
| Magnesium stearate | 2 mg |

A powdery mixture of the above formulation is compressed to provide tablets.

Formulation Example 3

Tablets (oral)

In each 120 mg per tablet

| | |
|---|---|
| Compound 5 | 1 mg |
| Lactose | 60 mg |
| Corn starch | 30 mg |
| Crystalline cellulose | 20 mg |
| Hydroxypropylcellulose | 7 mg |
| Magnesium stearate | 2 mg |

A powdery mixture of the above formulation is compressed to provide tablets.

INDUSTRIAL APPLICABILITY

As described above, the adenosine $A_2$ receptor agonist of the invention has hepatic damage inhibitory activity and is a safe compound with low toxicity, and is, therefore, useful as a prophylactic or therapeutic drug for hepatic damage in man and other mammals.

**Claims**

1. A pharmaceutical composition for prophylaxis or therapy of hepatic damage which comprises an adenosine $A_2$ receptor agonist as an active ingredient.

2. A pharmaceutical composition for prophylaxis or therapy of hepatic damage which comprises an adenosine $A_2$ receptor agonist having the following formula [1] with meeting the definition (a) or (b) below, an ester derivative in which the free hydroxyl group or groups are esterified in a pharmaceutically acceptable form, or a salt of said compound or ester derivative as an active ingredient.

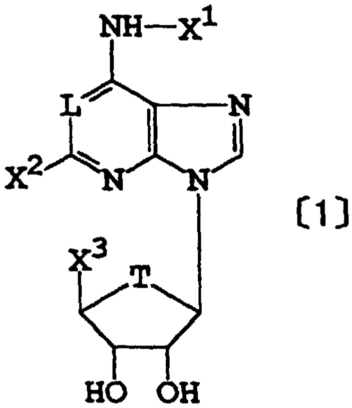

[1]

(a) Referring to formula [1], L represents N; T represents O; $X^1$ represents (1) hydrogen, (2) alkyl, (3) cycloalkyl, (4) aralkyl, (5) diarylalkyl (wherein one of the aryl moieties may be substituted by 1-3 same or different species of the substituent group $Y^8$ and the other aryl moiety being optionally substituted by 1-3 same or different species of the substituent group $Y^9$), or (6) arylheteroarylalkyl (wherein the aryl moiety may be substituted by 1-3 same or different species of the substituent group $Y^8$ and the heteroaryl moiety of which may be substituted by 1-3 same or different species of the substituent group $Y^9$); said substituent groups $Y^8$ and $Y^9$ are respectively selected from the group consisting of alkyl, halogen, alkoxy, alkylthio, trifluoromethyl, and tetramethylene;

$X^2$ represents hydrogen or any of the following substituent groups [i]-[iii]

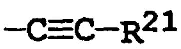

[i]

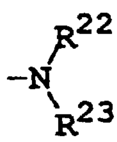

[ii]

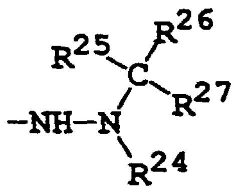

[iii]

wherein $R^{21}$ represents (1) cyano, (2) acyl, (3) carboxy, (4) alkoxycarbonyl, (5) alkenyl, (6) alkynyl, (7) aryl, (8) azido, (9) cycloalkyl which may be substituted by hydroxy, or (10) alkyl which may be substituted by the substituent $Y^1$ (the substituent $Y^1$ represents hydroxy, alkoxy, amino, carboxy, alkoxycarbonyl, alkenyl, alkynyl, aryl, azido, cyano or amino substituted by 1 or 2 same or different alkyl);

$R^{22}$ represents hydrogen or alkyl;

$R^{23}$ represents (1) cycloalkyl, (2) (cycloalkyl)alkyl wherein the cycloalkyl moiety may be substituted by the substituent $Y^2$ [the substituent $Y^2$ represents alkyl, hydroxy, alkylcarbonyloxy, alkoxy, or alkylenedioxy (wherein the two oxygen atoms are bound to either the same carbon atom or vicinal carbon atoms)], (3) aryl which may be substituted by 1-3 same or different species of the substituent group $Y^3$, (4) aralkyl (wherein the aryl moiety may be substituted by 1-3 species of the substituent group $Y^3$ and the alkyl moiety may be substituted by hydroxy), (5) arylcycloalkyl (wherein the aryl moiety may be substituted by 1-3 same or different species of the substituent group $Y^3$), (6) heteroaryl which may be substituted by 1-3 same or different species of the substituent group $Y^3$, (7) heteroarylalkyl (wherein the heteroaryl moiety may be substituted by 1-3 same or different species of the substituent group $Y^3$), (8) 9-fluorenyl, (9) 9-fluorenylalkyl, (10) 2-norbornyl, (11) bicycloheptylalkyl (wherein the bicycloheptyl moiety may be substituted by alkyl), (12) bicycloheptenylalkyl (wherein the bicycloheptenyl moiety may be substituted by alkyl), (13) adamantylalkyl, (14) cyclohexenylalkyl (wherein the cyclohexenyl moiety may be substituted by alkyl), (15) tetrahydropyranylalkyl, (16) tetrahydrothiopyranylalkyl, or

(17) the following substituent group,

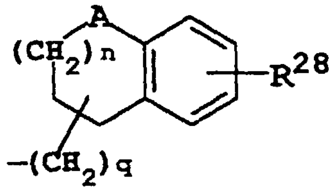

wherein A represents methylene, oxygen, or sulfur; n represents 0 or 1; q represents an integer of 0-2, $R^{28}$ represents hydrogen, alkyl, alkoxy, halogen or the substituent group -W-Z defined hereinafter.

The substituent group $Y^3$ is selected from the group consisting of (1) alkyl, (2) alkoxy, (3) alkylthio, (4) alkylcarbonyloxy, (5) halogen, (6) hydroxy, (7) benzyloxy (wherein the benzene moiety may be substituted by halogen), (8) tri-fluoromethyl, and (9) the substituent group -W-Z.

W represents (1) a single bond, (2) alkylene, (3) alkenylene, (4) thioalkylene, or (5) oxyalkylene. Z represents (1) cyano, (2) carboxy, (3) alkoxycarbonyl, (4) carbamoyl, (5) carbamoyl substituted by 1-2 same or different species of alkyl, or (6) carbamoyl substituted by alkyl which is substituted by $NHR^{29}$ ($R^{29}$ represents hydrogen, alkyl, alkenyl, acyl, or isothiocyano).

$R^{25}$, $R^{26}$, and $R^{27}$ may be the same or different and each represents hydrogen, alkyl, cycloalkyl, or aryl which may be substituted by the substituent group $Y^5$. The substituent group $Y^5$ represents alkyl, alkoxy, nitro, or amino which may be substituted by alkyl or phenyl,. $R^{24}$ represents hydrogen or alkyl; or $R^{24}$ and $R^{27}$ taken together represent a bond.

$X^3$ represents hydroxymethyl, alkoxymethyl, aminomethyl, azidomethyl, cyanomethyl, carboxy, alkoxycarbonyl, cycloalkyloxycarbonyl, $-CON(R^{32})(R^{33})$, $-CH_2NHSO_2R^{34}$, $-CH_2NHC(=O)R^{35}$, or $-CH_2NHC(=S)R^{36}$.

$R^{32}$ and $R^{33}$ may be the same or different and each represents hydrogen, aralkyl, cycloalkyl, or alkyl which may be substituted by hydroxy. $R^{34}$ represents alkyl or aryl which may be substituted by alkyl. $R^{35}$ and $R^{36}$ each represents hydrogen, alkyl, amino, or alkylamino.

Provided, however, that at least one of $X^1$ and $X^2$ represents hydrogen.

(b) Referring to formula [1], L represents N, N→O, or CH; T represents $CH_2$ or O; $X^1$ represents $-E-(G)_m-R^{50}$.

E represents (1) alkylene which may be substituted by 1-3 same or different species of $Y^6$, (2) cycloalkylene which may be substituted by 1-3 same or different species of $Y^6$, or (3) cycloalkenylene which may be substituted by 1-3 same or different species of $Y^6$. The substituent $Y^6$ is selected from the group consisting of alkyl, halogen, and alkoxy.

G represents $NR^{54}$, oxygen, or sulfur; m represents 0 or 1.

$R^{54}$ represents hydrogen, alkyl, aryl, or heteroaryl.

$R^{50}$ represents heteroaryl which may be substituted by 1-3 same or different species of $Y^7$. $Y^7$ represents (1) hydrogen, (2) hydroxy, (3) alkyl which may be substituted by hydroxy, (4) alkylthio, (5) mercaptoalkyl, (6) alkoxy, (7) alkoxyalkyl, (8) amino which may be substituted by alkyl, (9) carboxy, (10) acyl, (11) halogen, (12) carbamoyl which may be substituted by alkyl, (13) aryl, or (14) heteroaryl.

$X^2$ represents hydrogen.

$X^3$ represents $-CON(R^{51})(R^{52})$ or $-CH_2OR^{53}$.

$R^{51}$, $R^{52}$, and $R^{53}$ may be the same or different and each represents hydrogen, alkyl, aryl, or heteroaryl.

**3.** A pharmaceutical composition for prophylaxis or therapy of hepatic damage which comprises an adenosine $A_2$ receptor agonist selected from among the following compounds (1) through (16), inclusive of their salts, as an active ingredient:

(1) 1-[6-Amino-2-[(2-(4-(3-((2-aminoethyl)amino)-3-oxopropyl)phenyl)ethyl)amino]-9H-purin-9-yl]-1-deoxy-N-ethyl-β-D-ribofuranuronamide
(2) 2-[(2-Cyclohexylethyl)amino]adenosine
(3) 2-[(2-(1-Cyclohexen-1-yl)ethyl)amino]adenosine
(4) 2-(1-Octyn-1-yl)adenosine
(5) 1-[6-Amino-2-[(2-(4-(2-carboxyethyl)phenyl)ethyl)amino]-9H-purin-9-yl]-1-deoxy-N-ethyl-β-D-ribofuranuronamide
(6) 1-[6-Amino-2-[(2-(4-(2-carboxyethyl)phenyl)ethyl)amino]-9H-purin-9-yl]-1-deoxy-N-ethyl-β-D-ribofuranuronamide sodium salt
(7) 1-[6-Amino-2-(1-hexyn-1-yl)-9H-purin-9-yl)-1-deoxy-β-D-ribofuranuronamide
(8) 1-[6-Amino-2-(1-hexyn-1-yl)-9H-purin-9-yl]-1-deoxy-N-ethyl-β-D-ribofuranuronamide

(9) 4-(7-((1-((3-Chloro-2-thienyl)methyl)propyl)amino)-3H-imidazo [4,5-b]pyridin-3-yl]-N-ethyl-2,3-dihydroxycyclopentanecarboxamide
(10) 2-[((R)-1-Methyl-2-phenylethyl)amino]adenosine
(11) 2-[(Cyclohexylmethylene)hydrazino]adenosine
(12) 2-(Phenylamino)adenosine
(13) $N^6$-[2-(3,5-Dimethoxyphenyl)-2-(2-methylphenyl)-ethyl]adenosine
(14) 1-(6-Amino-9H-purin-9-yl)-1-deoxy-N-cyclopropyl-β-D-ribofuranuronamide
(15) Ethyl 1-(6-amino-9H-purin-9-yl)-1-deoxy-β-D-ribofuranuronate
(16) Adenosine

**4.** Use of an adenosine $A_2$ receptor agonist as defined in Claims 1-3 for the manufacture of drugs for prophylaxis or therapy of hepatic damage.

## INTERNATIONAL SEARCH REPORT

International application No.
PCT/JP98/02223

A. CLASSIFICATION OF SUBJECT MATTER
Int.Cl[6] A61K45/00, A61K31/70, A61K31/44

According to International Patent Classification (IPC) or to both national classification and IPC

B. FIELDS SEARCHED

Minimum documentation searched (classification system followed by classification symbols)
Int.Cl[6] A61K45/00, A61K31/70, A61K31/44

Documentation searched other than minimum documentation to the extent that such documents are included in the fields searched

Electronic data base consulted during the international search (name of data base and, where practicable, search terms used)
CA (STN), REGISTRY (STN), WPIDS (STN)

C. DOCUMENTS CONSIDERED TO BE RELEVANT

| Category* | Citation of document, with indication, where appropriate, of the relevant passages | Relevant to claim No. |
| --- | --- | --- |
| A | JP, 3-167197, A (The Wellcome Foundation Ltd.), 19 July, 1991 (19. 07. 91), Reference as a whole & EP, 421739, A | 1-4 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| 4 September, 1998 (04. 09. 98) | 16 September, 1998 (16. 09. 98) |
| Name and mailing address of the ISA/<br>Japanese Patent Office | Authorized officer |
| Facsimile No. | Telephone No. |

Form PCT/ISA/210 (second sheet) (July 1992)